# EUROPEAN PATENT SPECIFICATION

(11) **EP 2 461 803 B1**
(45) Date of publication and mention of the grant of the patent: **17.10.2018**
(21) Application number: 10807042.6
(22) Date of filing: 03.08.2010
(51) Int. Cl.: A61K 31/192, A61K 47/18, A61K 9/20, A61P 29/00

(54) **FAST-ACTING NAPROXEN COMPOSITION WITH REDUCED GASTROINTESTINAL EFFECTS**
SCHNELL WIRKENDE NAPROXENZUSAMMENSETZUNG MIT REDUZIERTER WIRKUNG AUF MAGEN UND DARM
COMPOSITION DE NAPROXÈNE À ACTION RAPIDE AVEC EFFETS GASTRO-INTESTINAUX RÉDUITS

(30) Priority: 03.08.2009 US 230964 P
(43) Date of publication of application: 13.06.2012
(73) Proprietor: Emisphere Technologies, Inc., Cedar Knolls, New Jersey 07927 (US)
(72) Inventor: NOVINSKI, Michael, Cedar Knolls, NJ 07927 (US); OSINSKI, Patrick, Cedar Knolls, NJ 07927 (US); HART, Nicholas, Cedar Knolls, NJ 07927 (US)
(74) Representative: Pons
(86) International application number: PCT/US2010/044279
(87) International publication number: WO 2011/017346

(56) References cited:
- US-A- 5 773 647
- US-A- 5 866 536
- US-A1- 2003 008 900
- US-A1- 2005 186 267
- US-A1- 2006 135 402
- US-A1- 2007 167 498
- US-A1- 2008 188 571
- US-A1- 2008 255 250
- US-A1- 2009 123 562
- B. G. CHARLES ET AL: "Comparativein vitro andin vivo bioavailability of naproxen from tablet and caplet formulations", BIOPHARMACEUTICS & DRUG DISPOSITION, vol. 15, no. 2, 1 March 1994 (1994-03-01), pages 121-128, XP055074480, ISSN: 0142-2782, DOI: 10.1002/bdd.2510150204
- HESS S ET AL: "Investigation of the enhancing mechanism of sodium N-[8-(2-hydroxybenzoyl)amino]caprylate effect on the intestinal permeability of polar molecules utilizing a voltage clamp method", EUROPEAN JOURNAL OF PHARMACEUTICAL SCIENCES, ELSEVIER, AMSTERDAM, NL, vol. 25, no. 2-3, 1 June 2005 (2005-06-01) , pages 307-312, XP027803754, ISSN: 0928-0987 [retrieved on 2005-06-01]
- LEONE-BAY A ET AL: "ORAL DELIVERY OF SODIUM CROMOLYN: PRELIMINARY STUDIES IN VIVO AND IN VITRO", PHARMACEUTICAL RESEARCH, KLUWER ACADEMIC PUBLISHERS, NEW YORK, NY, US, vol. 13, no. 2, 1 January 1996 (1996-01-01), pages 222-226, XP008030577, ISSN: 0724-8741, DOI: 10.1023/A:1016034913181

## Description

### Field of the Invention

The present invention relates to oral pharmaceutical compositions containing naproxen or a pharmaceutically acceptable salt thereof and a delivery agent.

### Background of the Invention

- D1: US 2005/186267 A1 (THOMPSON DIANE O [US] ET AL) 25 August 2005 (2005-08-25)
- D2: B. G. CHARLES ET AL: "Comparativein vitro and in vivo bioavailability of naproxen from tablet and caplet formulations", BIOPHARMACEUTICS & DRUG DISPOSITION, vol. 15, no. 2, 1 March 1994 (1994-03-01), pages 121-128, ISSN: 0142-2782, DOI: 10.1002/bdd.2510150204
- D3: HESS S ET AL: "Investigation of the enhancing mechanism of sodium N-[8-(2-hydroxybenzoyl)amino]caprylate effect on the intestinal permeability of polar molecules utilizing a voltage clamp method", EUROPEAN JOURNAL OF PHARMACEUTICAL SCIENCES, ELSEVIER, AMSTERDAM, NL, vol. 25, no. 2-3, 1 June 2005 (2005-06-01), pages 307-312, ISSN: 0928-0987
- D4: LEONE-BAY A ET AL: "ORAL DELIVERY OF SODIUM CROMOLYN: PRELIMINARY STUDIES IN VIVO AND IN VITRO", PHARMACEUTICAL RESEARCH, KLUWER ACADEMIC PUBLISHERS, NEW YORK, NY, US, vol. 13, no. 2, 1 January 1996 (1996-01-01), pages 222-226, ISSN: 0724-8741, DOI: 10.1023/A:1016034913181

Naproxen is a propionic acid derivative related to the arylacetic acid group of nonsteroidal anti-inflammatory drugs. It has been widely used to reduce swelling and to treat pain, including dental pain, headache, painful monthly periods, painful joint and muscular problems such as arthritis, tendinitis, bursitis, and gout.

US 2005/186267 discloses compositions in capsules comprising an active ingredient and possibly a permeation enhancer. Naproxen is mentioned as possible active ingredient and SNAC or SNAD as possible permeation enhancers.

Naproxen is a weel-known NSAID and its pharmacokinetics have been studied extensively (e.g. in B.G. Charles et al: Biopharmaceutics & Drug Disposition 1994, 15, pg. 121-128.

SNAC has been described as delivery agent for biomolecules and for polar low molecular organic compounds (see Hess et al European Journal of Pharmaceuticals Sciences 2005, 25, pgs. 307-312 and Lenoe-Bay et al Pharmaceutical Research, Kluwer Academic Publishers, New York 1996, 13, pg. 222-226). Other known delivery agents have been described (see for example, US5773647, US2008255250, US5866536 and US2003008900.

Naproxen is rapidly and completely absorbed from the gastrointestinal tract with an in vivo bioavailability of 95%. The elimination half-life of Naproxen ranges from 12 to 17 hours. Steady-state levels of Naproxen are reached in 4 to 5 days, and the degree of Naproxen accumulation is consistent with this half-life. However, it takes hours to reach peak plasma levels of Naproxen. When given as Naproxen suspension, peak plasma levels of Naproxen are attained in 1 to 4 hours. When given as Naproxen tablets, peak plasma levels of Naproxen are attained in 2 to 4 hours. Moreover, Naproxen causes an increased risk of serious gastrointestinal adverse events including bleeding, ulceration, and perforation of the stomach or intestines, which can be fatal. These events can occur at any time during use and without warning symptoms. Elderly patients are at greater risk for serious gastrointestinal events. There is a need to develop Naproxen formulations which can reach peak plasma levels to provide quick relief and with lower gastrointestinal adverse events.

### Summary of the Invention

The present invention is an oral pharmaceutical composition (e.g., a tablet or suspension) comprising Naproxen and at least one delivery agent. The pharmaceutical composition provides a faster onset of action of Naproxen to a subject (e.g., a human subject) than a similar composition without the delivery agent, thereby providing faster pain relief and reducing the risk of developing gastric ulcers. Furthermore, the pharmaceutical compositions of the present invention are significantly smaller than fast acting liquid gel capsules containing the same amount of naproxen. The reduced size can improve patient comfort and compliance.

The present invention is an oral pharmaceutical composition comprising Naproxen and at least one delivery agent, being SNAC. In one preferred embodiment, the oral pharmaceutical composition provides, upon ingestion to a subject (e.g., a healthy human subject), a shortened time period for reaching peak plasma Naproxen levels, compared to a similar composition without the delivery agent.

In one embodiment, the oral pharmaceutical composition includes from about 50 to about 600 mg of Naproxen (calculated on the weight basis of the naproxen base). In other embodiments the oral pharmaceutical composition includes from about 100 to about 400 mg, from about 150 to about 300 mg, or from about 150 to about 250 mg of Naproxen (calculated on the weight basis of the naproxen base). In yet another embodiment, the oral pharmaceutical composition includes about 200 mg of Naproxen (calculated on the weight basis of the naproxen base). For instance, the oral pharmaceutical composition may include about 220 mg of naproxen sodium (equivalent to 200 mg of naproxen base).

The oral pharmaceutical composition may further include another analgesic, such as a 5-HT₁ agonist (e.g., sumatriptan or a pharmaceutically acceptable salt thereof, such as sumatriptan succinate). In one embodiment, the oral pharmaceutical composition includes from about 25 to about 100 mg sumatriptan (e.g., 85 mg) and from about 225 to about 825 mg Naproxen (e.g., 500 mg naproxen sodium).

The oral pharmaceutical composition may further include a proton pump inhibitor, such as omeprazole, esomeprazole, lansoprazole, or any combination thereof. The proton pump inhibitor may be incorporated at a dose sufficient to further reduce the risk of developing gastric ulcers in subjects at risk of developing non-steroidal anti-inflammatory drug (NSAID) associated gastric ulcers.

Another embodiment is a method of providing rapid oral delivery of naproxen to a subject by orally administering an oral pharmaceutical composition of the present invention.

Yet another embodiment is a method of reducing gastrointestinal adverse events caused by oral ingestion of Naproxen in a human subject. The method includes administering an oral pharmaceutical composition of the present invention.

Yet another embodiment is a method of reducing gastrointestinal adverse events caused by oral ingestion of a Naproxen formulation in a human subject. The method includes discontinuing administration of the Naproxen formulation and initiating treatment with an oral pharmaceutical composition of the present invention (e.g., administering an oral pharmaceutical composition of the present invention).

Yet another embodiment is a method of treating pain in a subject in need thereof. The method includes administering an oral pharmaceutical composition of the present invention.

Yet another embodiment is a method of providing relief of the signs and symptoms of rheumatoid arthritis, osteoarthritis, ankylosing spondylitis, juvenile arthritis, tendonitis, bursitis, or acute gout in a subject in need thereof by administering one or more oral pharmaceutical compositions of the present invention. Administration of an effective amount of Naproxen can be achieved with a single composition containing an effective amount of Naproxen or by administration of two or more compositions.

Yet another embodiment is a method of treating pain (e.g., acute pain) or primary dysmenorrheal in a subject in need thereof by administering one or more oral pharmaceutical compositions of the present invention.

Yet another embodiment is a method of treating migraine attacks in a subject in need thereof by administering one or more oral pharmaceutical compositions of the present invention. In one embodiment, the oral pharmaceutical composition further includes another analgesic, such as a 5-HT₁ agonist (e.g., sumatriptan or a pharmaceutically acceptable salt thereof, such as sumatriptan succinate).

In each of the aforementioned methods, oral pharmaceutical compositions of the present invention may be administered once or twice daily to provide a dose of from about 200 to about 600 mg naproxen at each administration. For acute gout, an initial dose of from about 600 to about 1000 mg naproxen (e.g., 750 or 825 mg) followed by from about 200 to about 350 mg naproxen (e.g., 250 or 275 mg) every 8 hours until the attack has subsided.

In one embodiment, one or two oral pharmaceutical compositions of the present invention (e.g., oral pharmaceutical compositions containing 200 mg of Naproxen, for instance 220 mg naproxen sodium) are administered every 8 to 12 hours. In one preferred embodiment, no more than three oral pharmaceutical compositions of the present invention are administered in a 24 hour period.

### Detailed Description of the Invention

### Definitions

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviations, per practice in the art. Alternatively, "about" with respect to the compositions can mean a range of up to 10%, preferably up to 5%.

The terms "alkyl", "alkenyl", "alkoxy", "alkylene", "alkenylene", "alkyl(arylene)", and "aryl(alkylene)" include, but are not limited to, linear and branched alkyl, alkenyl, alkoxy, alkylene, alkenylene, alkyl(arylene), and aryl(alkylene) groups, respectively.

The phrase "pharmaceutically acceptable" refers to compounds or compositions that are physiologically tolerable and do not typically produce an allergic or similar untoward reaction, such as gastric upset, dizziness and the like, when administered to a mammal.

As used herein, the term "peak plasma level" means the maximum concentration reached in the plasma of a mammal, such as a human subject.

The term "bioavailability" refers to the rate and extent to which the active ingredient or active moiety is absorbed from a drug product and becomes systematically available.

The term "polymorph" refers to crystallographically distinct forms of a substance.

The term "hydrate" as used herein includes, but is not limited to, (i) a substance containing water combined in the molecular form and (ii) a crystalline substance containing one or more molecules of water of crystallization or a crystalline material containing free water.

The term "SNAC" as used herein refers to N-(8-[2-hydroxybenzoyl]-amino) caprylic acid and pharmaceutically acceptable salts thereof, including its monosodium and disodium salt. The term "SNAC free acid" refers to N-(8-[2-hydroxybenzoyl]-amino)caprylic acid. Unless otherwise noted, the term "SNAC" refers to all forms of SNAC, including all amorphous and polymorphic forms of SNAC, such as SNAC trihydrate and those described in U.S. Serial No. 11/568,753 and PCT Application No. PCT/US2005/016126, both of which are hereby incorporated by reference.

The term "SNAD" as used herein refers to N-(10-[2-hydroxybenzoyl]-amino)decanoic acid and pharmaceutically acceptable salts thereof, including its monosodium salt. Unless otherwise noted, the term "SNAD" refers to all forms of SNAD, including all amorphous and polymorphic forms of SNAD.

The term "4-CNAB" as used herein refers to 4-[(4-chloro-2-hydroxybenzoyl)amino]-butanoic acid (also known as 4-[(2-hydroxy-4-chlorobenzoyl)amino]butanoate) and pharmaceutically acceptable salts thereof, including its sodium salt (e.g., monosodium salt). Unless otherwise noted, the term "4-CNAB" refers to all forms of 4-CNAB, including all amorphous and polymorphic forms of 4-CNAB. The term "sodium 4-CNAB" and "mono-sodium 4-CNAB" refer to monosodium 4-[(2-hydroxy-4-chlorobenzoyl)amino]butanoate, including anhydrous, monohydrate, and isopropanol solvates thereof and amorphous and polymorphic forms thereof such as those described in U.S. Patent Nos. 7,227,033, 7,208,178, 7,462,368, and 7,420,085.

The term "5-CNAC" as used herein refers to 8-(N-2-hydroxy-5-chlorobenzoyl)amino-caprylic acid and pharmaceutically acceptable salts thereof, including its monosodium and disodium salt. The term "5-CNAC free acid" refers to 8-(N-2-hydroxy-5-chlorobenzoyl)aminocaprylic acid. Unless otherwise noted, the term "5-CNAC" refers to all forms of 5-CNAC, including all amorphous and crystalline forms of 5-CNAC, such as crystalline forms of the disodium salt of 5-CNAC, such as those described in U.S. Patent Publication No. 2008-0269108, which is hereby incorporated by reference.

The term "4-MOAC" as used herein refers to 8-(N-2-hydroxy-4-methoxybenzoyl)-aminocaprylic acid and pharmaceutically acceptable salts thereof, including its monosodium and disodium salt. The term "4-MOAC free acid" refers to 8-(N-2-hydroxy-4-methoxybenzoyl)-aminocaprylic acid. Unless otherwise noted, the term "4-MOAC" refers to all forms of 4-MOAC

The term "delivery agent" refers to any of the delivery agent compounds disclosed or incorporated by reference herein.

The terms "2-OH-Ar" or "2-HO-Ar", as used in formulas 1 and 2 refers to an aryl group that is substituted with a hydroxy group at the 2 position.

The term "subject" includes mammals and in particular humans.

### Delivery Agent Compounds

The delivery agent is SNAC (the free acid or a pharmaceutically acceptable thereof). In one embodiment, the delivery agent is a sodium salt of SNAC. In another embodiment, the delivery agent is the monosodium salt of SNAC and can be, for example, any of the solid state forms of monosodium SNAC disclosed in U.S. Application No. 11568,753 and PCT Application No. PCT/US2005/016126, both of which are hereby incorporated by reference. In yet another embodiment, the delivery agent is the disodium salt of SNAC.

The delivery agent compounds depicted as carboxylic acids may be in the form of the carboxylic acid or salts thereof. Suitable salts include, but are not limited to, organic and inorganic salts, for example alkali-metal salts, such as sodium (e.g., monosodium and disodium salts), potassium and lithium; alkaline-earth metal salts, such as magnesium, calcium or barium; ammonium salts; basic amino acids, such as lysine or arginine; and organic amines, such as dimethylamine or pyridine. Preferably, the salts are sodium salts. The salts may be mono-or multi-valent salts, such as monosodium salts and di-sodium salts. The salts may also be solvates, including ethanol solvates, and hydrates.

The delivery agent compounds depicted as amines may be in the form of the free amine or salts thereof. Suitable salts include, but are not limited to, organic and inorganic salts, for example sodium salts, sulfate salts, hydrochloride salts, phosphate salts, fluoride salts, carbonate salts, tartrate salts, oxalates, oxides, formates, acetate or citrate.

Salts of the delivery agent compounds of the present invention may be prepared by methods known in the art. For example, sodium salts may be prepared by dissolving the delivery agent compound in ethanol and adding aqueous sodium hydroxide.

Where the delivery agent has an amine moiety and a carboxylic acid moiety, poly amino acids and peptides comprising one or more of these compounds may be used. An amino acid is any carboxylic acid having at least one free amine group and includes naturally occurring and synthetic amino acids. Poly amino acids are either peptides (which are two or more amino acids joined by a peptide bond) or are two or more amino acids linked by a bond formed by other groups which can be linked by, e.g., an ester or an anhydride linkage. Peptides can vary in length from dipeptides with two amino acids to polypeptides with several hundred amino acids. One or more of the amino acids or peptide units may be acylated or sulfonated.

The delivery agent compound may be purified by recrystallization or by fractionation on one or more solid chromatographic supports, alone or linked in tandem. Suitable recrystallization solvent systems include, but are not limited to, acetonitrile, methanol, ethanol, ethyl acetate, heptane, water, tetrahydrofuran, and combinations thereof. Fractionation may be performed on a suitable chromatographic support such as alumina, using methanol/n-propanol mixtures as the mobile phase; reverse phase chromatography using trifluoroacetic acid/acetonitrile mixtures as the mobile phase; and ion exchange chromatography using water or an appropriate buffer as the mobile phase. When anion exchange chromatography is performed, preferably a 0-500 mM sodium chloride gradient is employed.

### Naproxen

Naproxen means 2-(6-methoxynaphthalen-2-yl)propanoic acid and pharmaceutically acceptable salts thereof, including its sodium salt. The term "Naproxen free acid" refers to 2-(6-methoxynaphthalen-2-yl)propanoic acid. Unless otherwise noted, the term "Naproxen" refers to all forms of Napraxen, including all amorphous and crystalline forms of Naproxen.

Solid pharmaceutical compositions may be in the form of tablets, capsules (including hard and soft gelatin capsules), and particles, such as powders and sachets. An oral tablet is a preferred dosage form of the present invention. Solid dosage forms may be prepared by mixing the solid form of the delivery agent with the solid form of the incretin hormone. Alternately, a solid may be obtained from a solution of delivery agent and incretin hormone by methods known in the art, such as freeze-drying (lyophilization), precipitation, crystallization and solid dispersion.

The pharmaceutical compositions can include anyone or combination of excipients, diluents, disintegrants, lubricants, fillers, plasticizers, colorants, flavorants, taste-masking agents, sugars, sweeteners and salts.

In one embodiment, the weight ratio of delivery agent to Naproxen is from about 1:8 to about 2:1. In another embodiment, the weight ratio of delivery agent to Naproxen is from about 1:6 to about 1.5:1, from about 1:4 to about 1:1.25, or from about 1:4.5 to about 1:1.

In one embodiment, the delivery agent is SNAC, SNAD, 5-CNAC, 4-MOAC, or 4-CNAB and the weight ratio of delivery agent and Naproxen is between 1:5 and 20:1, preferably between 1:3 and 15:1, more preferably between 1:1 and 10:1, and the most preferably between 2:1 and 5:1.

In yet another embodiment, the delivery agent is SNAC, SNAD, 5-CNAC, 4-MOAC, or 4-CNAB, the Naproxen is naproxen sodium, and the weight ratio of SNAC, SNAD or 4-CNAB to naproxen sodium is about 1:4.4, about 1:2.2, or about 1:1.1.

In yet another embodiment, the delivery agent is the monosodium salt of SNAC, the Naproxen is naproxen sodium, and the weight ratio of the monosodium salt of SNAC to naproxen sodium is about 1:4.4, about 1:2.2, or about 1:1.1.

In another embodiment, the pharmaceutical composition contains from about 25 mg to about 500 mg of Naproxen and from about 50 mg to about 600 mg of delivery agent. Preferably, the pharmaceutical composition, upon oral ingestion to a human, provides peak plasma levels of Naproxen in 60 minutes or less, more preferably in 45 minutes or less, and the most preferably in 30 minutes or less.

In yet another embodiment, the pharmaceutical composition includes from about 25 mg to about 500 mg of Naproxen and from about 50 mg to about 600 mg of the monosodium salt of SNAC. For example, the pharmaceutical composition may include from about 150 to about 300 mg of Naproxen (preferably naproxen sodium) (calculated based on weight of naproxen base) and from about 50 to about 200 mg of SNAC (preferably the monosodium salt of SNAC).

The pharmaceutical compositions are useful for treating pains with reduced gastrointestinal adverse events caused by oral ingestion of Naproxen in a human subjects. Preferably the gastrointestinal adverse events are reduced by more than 20% as compared with administration of Naproxen alone, more preferably by more than 40% and the most preferably by more than 60%.

### Example 1

Naproxen tablets are made and each tablet contains 110 mg of Naproxen and 250 mg of SNAC. These tablets reach peak Naproxen levels at much shorter time as compared with Naproxen tablets that do not contain SNAC.

All publications, patents, and patent applications cited herein are hereby incorporated by reference.

## Claims

1. A tablet comprising (a) Naproxen and (b) at least one delivery agent selected from N-(8-[2-hydroxybenzoyl]amino)caprylic acid, and pharmaceutically acceptable salts thereof, wherein the weight ratio of said delivery agent and said Naproxen is between 1:5 and 20:1.

2. The tablet of claim 1, wherein the weight ratio of said N-(8-[2-hydroxybenzoyl] amino)caprylic acid, or a pharmaceutically acceptable salt thereof, and said Naproxen is between 2:1 and 5:1.

3. The tablet of claim 1, wherein said tablet contains from about 25 mg to about 500 mg of Naproxen.

4. The tablet of claim 1, wherein said tablet contains from about 50 mg to about 600 mg of said delivery agent.

5. A tablet of any one of claims 1 to 4, for its use as a medicament.

6. A tablet of any one of claims 1 to 4, for its use as a medicament for treating pain, wherein the tablet comprises from about 150 to about 300 mg of naproxen or a pharmaceutically acceptable salt thereof (calculated on the weight basis of naproxen base) and from about 50 to about 200 mg of N-(8-[2-hydroxybenzoyl]amino)caprylic acid or a pharmaceutically acceptable salt thereof.

7. The tablet of claim 6, which comprises about 220 mg naproxen sodium and from about 50 to about 200 mg of a monosodium salt of N-(8-[2-hydroxybenzoyl]amino)caprylic acid.

8. The tablet of claim 1, which comprises monosodium salt N-(8-[2-hydroxybenzoyl] amino)caprylic acid and naproxen sodium in a weight ratio of about 1:1.1.

9. The tablet of claim 1, wherein the weight ratio of said delivery agent and said Naproxen is between 1:3 and 15:1.

10. The tablet of claim 1, wherein the weight ratio of said delivery agent and said Naproxen is between 1:1 and 10:1.

## Patentansprüche

1. Tablette, welche (a) Naproxen und (b) mindestens ein Abgabemittel, welches aus N-(8-[2-Hydroxybenzoyl]amino)caprylsäure und pharmazeutisch verträglichen Salzen davon ausgewählt ist, umfasst, wobei das Gewichtsverhältnis des Abgabemittels und des Naproxens zwischen 1:5 und 20:1 liegt.

2. Tablette nach Anspruch 1, wobei das Gewichtsverhältnis der N-(8-[2-Hydroxybenzoyl] amino)caprylsäure oder eines pharmazeutisch verträglichen Salzes davon, und des Naproxens zwischen 2:1 und 5:1 liegt.

3. Tablette nach Anspruch 1, wobei die Tablette etwa 25 mg bis etwa 500 mg Naproxen enthält.

4. Tablette nach Anspruch 1, wobei die Tablette etwa 50 mg bis etwa 600 mg des Abgabemittels enthält.

5. Tablette nach einem der Ansprüche 1 bis 4 zur Verwendung als ein Arzneimittel.

6. Tablette nach einem der Ansprüche 1 bis 4 zur Verwendung als ein Arzneimittel zur Behandlung von Schmerzen, wobei die Tablette etwa 150 bis etwa 300 mg Naproxen oder eines pharmazeutisch verträglichen Salzes davon (berechnet auf der Gewichtsbasis von Naproxen) und etwa 50 bis etwa 200 mg N-(8-[2-Hydroxybenzoyl]amino)caprylsäure oder eines pharmazeutisch verträglichen Salzes davon umfasst.

7. Tablette nach Anspruch 6, welche etwa 220 mg Naproxen-Natrium und etwa 50 bis etwa 200 mg eines Mononatriumsalzes von N-(8-[2-Hydroxybenzoyl]amino)caprylsäure umfasst.

8. Tablette nach Anspruch 1, welche Mononatriumsalz von N-(8-[2-Hydroxybenzoyl] amino)caprylsäure und Naproxen-Natrium in einem Gewichtsverhältnis von etwa 1:1,1 umfasst.

9. Tablette nach Anspruch 1, wobei das Gewichtsverhältnis des Abgabemittels und des Naproxens zwischen 1:3 und 15:1 liegt.

10. Tablette nach Anspruch 1, wobei das Gewichtsverhältnis des Abgabemittels und des Naproxens zwischen 1:1 und 10:1 liegt.

## Revendications

1. Comprimé comprenant (a) du Naproxène et (b) au moins un agent de délivrance choisi dans l'acide N-(8-[2-hydroxybenzoyl]amino)caprylique, et ses sels pharmaceutiquement acceptables de celui-ci, dans lequel le rapport de poids dudit agent de délivrance et dudit Naproxène est entre 1:5 et 20:1.

2. Comprimé selon la revendication 1, dans lequel le rapport de poids dudit acide N-(8-[2-hydroxybenzoyl] amino)caprylique, ou un sel pharmaceutiquement acceptable de celui-ci, et dudit Naproxène est entre 2:1 et 5:1.

3. Comprimé selon la revendication 1, dans lequel ledit comprimé contient entre 25 mg environ et 500 mg environ de Naproxène.

4. Comprimé selon la revendication 1, dans lequel ledit comprimé contient entre 50 mg environ et 600 mg environ dudit agent de délivrance.

5. Comprimé selon l'une quelconque des revendications 1 à 4, pour son utilisation comme un médicament.

6. Comprimé selon l'une quelconque des revendications 1 à 4, pour son utilisation comme un médicament pour le traitement de la douleur, dans lequel le comprimé comprend entre 150 environ et 300 mg environ de naproxène ou un sel pharmaceutiquement acceptable de celui-ci (calculé sur la base pondérale de naproxène de base) et entre 50 environ et 200 mg environ d'acide N-(8-[2-hydroxybenzoyl]amino)caprylique ou un sel pharmaceutiquement acceptable de celui-ci.

7. Comprimé selon la revendication 6, qui comprend 220 mg environ de naproxène sodique et entre 50 environ et 200 mg environ d'un sel monosodique d'acide N-(8-[2-hydroxybenzoyl]amino)caprylique.

8. Comprimé selon la revendication 1, qui comprend le sel monosodique d'acide N-(8-[2-hydroxybenzoyl]amino)caprylique et le naproxène sodique dans un rapport de poids de 1:1.1 environ.

9. Comprimé selon la revendication 1, dans lequel le rapport de poids dudit agent de délivrance et dudit Naproxène est entre 1:3 et 15:1.

10. Comprimé selon la revendication 1, dans lequel le rapport de poids dudit agent de délivrance et dudit Naproxène est entre 1:1 et 10:1.
